Europäisches Patentamt

European Patent Office

Office européen des brevets

(19)

(11) Veröffentlichungsnummer: **0 537 492 B1**

## (12) EUROPÄISCHE PATENTSCHRIFT

(45) Veröffentlichungstag der Patentschrift: **17.05.95**

(21) Anmeldenummer: **92115888.7**

(22) Anmeldetag: **17.09.92**

Die Akte enthält technische Angaben, die nach dem Eingang der Anmeldung eingereicht wurden und die nicht in dieser Patentschrift enthalten sind.

(51) Int. Cl.6: **C07D 487/14**, //(C07D487/14, 241:00,239:00,235:00)

(54) **Stabile Salze von 5,10-Methylentetrahydrofolsäure.**

(30) Priorität: **15.10.91 CH 3019/91**

(43) Veröffentlichungstag der Anmeldung:
**21.04.93 Patentblatt 93/16**

(45) Bekanntmachung des Hinweises auf die Patenterteilung:
**17.05.95 Patentblatt 95/20**

(84) Benannte Vertragsstaaten:
**AT BE CH DE DK ES FR GB GR IE IT LI LU MC NL PT SE**

(56) Entgegenhaltungen:
**EP-A- 0 348 641**
**EP-A- 0 495 204**
**US-A- 3 468 886**

(73) Patentinhaber: **EPROVA Aktiengesellschaft**
**Im Laternenacker 5**
**CH-8200 Schaffhausen (CH)**

(72) Erfinder: **Müller, Hans Rudolf**
**Beckenwäldli 18**
**CH-8207 Schaffhausen (CH)**
Erfinder: **Ulmann, Martin**
**Steigstrasse 36**
**CH-8447 Dachsen (CH)**
Erfinder: **Conti, Josef**
**Winkelriedstrasse 22**
**CH-8203 Schaffhausen (CH)**

## Beschreibung

Die Erfindung betrifft neue stabile Salze von 5,10-Methylen-N-[4-[[(2-amino-1,4,5,6,7,8-hexahydro-4-oxo-(6R)-, (6R,S)- oder (6S)-pteridinyl)-methyl]amino]-benzol]-L-glutaminsäure - im folgenden kurz 5,10-Methylen-(6R)-, (6R,S)- oder (6S)-tetrahydrofolsäure genannt - mit Schwefelsäure oder einer Sulfonsäure sowie deren Herstellung und Verwendung.

Tetrahydrofolate sind die biologisch aktiven Formen der Folsäure (Folsäure-Co-Faktoren).

Als Arzneimittel werden Tetrahydrofolate vorwiegend als Calciumsalz der 5-Formyl-5,6,7,8-tetrahydrofolsäure [Leucovorin] verwendet, z.B. zur Verstärkung des therapeutischen Effektes von 5-Fluoruracil oder z. B. als Rescue-Subsanz beim Einsatz von Methotrexat in der Krebstherapie.

Im Organismus wird 5-Formyl-(6S)-tetrahydrofolsäure in 5,10-Methylen-(6R)-tetrahydrofolsäure umgewandelt, welche als Co-Faktor mit aus 5-Fluoruracil (5-FU) gebildetem 5-Fluor-Desoxyuridinmonophosphat (5-F-dUMP) und Thymidylat-Synthetase (TS) einem cytostatisch wirksamen covalenten ternären Komplex: 5-F-dUMP/TS/Methylentetrahydrofolsäure bildet. Siehe dazu: W.A. Bleyer, Cancer, March 15 Supplement 1989: S. 995-1007 sowie E.L.R. Stokstad, Folic Acid Metabolism in Health and Disease 1990 - (Wiley-Liss Inc), Seite 9.

Es wäre daher vor Vorteil, anstelle von Leucovorin (5-Formyltetrahydrofolsäure) direkt den Co-Faktor 5,10-Methylentetrahydrofolsäure zu verwenden. Bisher scheiterte dieses Unterfangen an der nicht ausreichenden Reinheit und Stabilität von 5,10-Methylentetrahydrofolsäure und deren Salze. Vergleiche dazu EP-A-0 409 125. Sowohl die in EP-A-348 641 beschriebenen Salze der 5,10-Methenylentetrahydrofolsäure, wie auch das von US-3 468 886 beanspruchte (6R,S)-Tetrahydrohomofolsäure-Sulfat können aufgrund ihrer sich zu den erfindungsgemässen Salzen in wesentlichen Merkmalen unterscheidenden Struktur und ihres darauf basierenden verschiedenen Verhaltens zur Lösung der Aufgabe nicht herangezogen werden. Die Frage der Stabilität hat sich weder beim EP-A-348 641 noch bei US-3 468 886 gestellt.

Es wurde nun überraschend gefunden, dass sich Salze von 5,10-Methylen-(6R)-, (6R,S)- und (6S)-tetrahydrofolsäure nach Umsetzung der entsprechenden diastereomeren Form der 5,10-Methylentetrahydrofolsäure insbesondere mit Schwefelsäure oder aber auch mit Sulfonsäuren in bisher nie erreichter Reinheit und ausgezeichneter Stabilität erhalten lassen.

Die diastereomeren Formen der 5,10-Methylentetrahydrofolsäure werden bevorzugt aus dem durch die schweizerische Patentanmeldung Nr. 108 vom 16.01.1991 leicht zugänglich gewordenen (6S)-, (6R,S)- und (6R)-Tetrahydrofolsäure•Sulfat oder Sulfonsäure-Salz in situ durch Umsetzung mit Formaldehyd hergestellt. Dabei entsteht aus der natürlichen (6S)-Tetrahydrofolsäure die natürliche 5,10-Methylen-(6R)-tetrahydrofolsäure. Das rührt daher, dass die absolute Konfiguration am C-6 der natürlichen Tetrahydrofolsäure nach J.C. Fontecilla-Camps et al, J. Amer. chem. Soc. 101 - (20), 6114-15 (1979) gemäss der Sequenz-Regel mit S zu spezifizieren ist und diejenige an C-6 der natürlichen 5,10-Methylentetrahydrofolsäure mit R. Siehe R. Kalbermatten et al, Helv. chim. Acta 64 - (8), 2627 (1981), Fussnote 4.

Die Umsetzung mit Schwefelsäure bzw. einer Sulfonsäure wird in Wasser und/oder in Gegenwart einer niedrigen aliphatischen wasserlöslichen Carbonsäure oder eines niedrigen Alkohols vorgenommen, wobei der Zusatz eines Alkohols auch zur Ausscheidung des gebildeten Sulfates oder Sulfonates dient.

Die erhaltenen Produkte sind in fester Form bei Raumtemperatur praktisch unbeschränkt stabil. Sie sind geeignet als Bestandteile von oralen Arzneimittelformen oder als Ausgangsmaterial zur Herstellung von parenteralen Arzneimittelformen. Sowohl die oralen als auch die parenteralen Arzneimittelformen sind z.B. zur Krebstherapie, zur Behandlung von bestimmten Anämie-Formen, von Autoimmunkrankheiten und von neuralen Störungen geeignet.

Gegenstand der Erfindung sind demnach die Salze von (6R)-, (6R,S)- und (6S)-5,10-Methylentetrahydrofolsäure mit Schwefelsäure oder einer Sulfonsäure.

Bevorzugte Verbindungen sind:
5,10-Methylen-(6R)-tetrahydrofolsäure•Sulfat,
5,10-Methylen-(6R,S)-tetrahydrofolsäure•Sulfat,
5,10-Methylen-(6S)-tetrahydrofolsäure•Sulfat,
5,10-Methylen-(6R)-, (6R,S)-, (6S)-tetrahydrofolsäure•Benzolsulfonat,
5,10-Methylen-(6R)-, (6R,S)-, (6S)-tetrahydrofolsäure•Toluol-4-sulfonat sowie
5,10-Methylen-(6R)-, (6R,S)-, (6S)-tetrahydrofolsäure•Methansulfonat.

Ein weiterer Gegenstand der Erfindung ist das Verfahren zur Herstellung der Salze von 5,10-Methylen-(6R)-, (6R,S)- und (6S)-tetrahydrofolsäure mit Schwefelsäure oder einer Sulfonsäure, welches dadurch gekennzeichnet ist, dass man die entsprechende diastereomere Form der 5,10-Methylentetrahydrofolsäure mit Schwefelsäure oder einer Sulfonsäure umsetzt.

Diese Umsetzung wird vorzugsweise mit in situ hergestellter 5,10-Methylentetrahydrofolsäure vorgenommen. Die Umsetzung erfolgt in einem Lösungsmittel bestehend aus Wasser oder einem mit Wasser mischbaren organischen Lösungsmittel wie einer niedrigen aliphatischen Carbonsäure oder einem niedrigen Alkohol.

Als Salzbildner kommen bevorzugt neben der Schwefelsäure in Betracht Benzolsulfonsäure, eine Toluolsulfonsäure, Xylolsulfonsäure, Nitrobenzolsulfonsäure, Chlorbenzolsulfonsäure, Nitrotoluolsulfonsäure, Naphthalinsulfonsäure, substituierte Naphthalinsulfonsäure, eine Camphersulfonsäure, Phenylmethansulfonsäure, Methansulfonsäure, Ethansulfonsäure, eine Propansulfonsäure oder Butansulfonsäure.

Als Lösungsmittel für die Isolierung der Salze von 5,10-Methylentetrahydrofolsäure wird Wasser, eine niedrige aliphatische wasserlösliche Carbonsäure wie Essigsäure, Milchsäure, Ameisensäure, ein wasserlöslicher Alkohol wie Methanol, Ethanol, Isopropanol oder ein Gemisch derselben eingesetzt.

Die Erfindung betrifft auch die Verwendung der stabilen Salze von 5,10-Methylen-(6R)-, (6R,S)- oder (6S)-tetrahydrofolsäure mit Schwefelsäure oder einer Sulfonsäure als Bestandteil und/oder Ausgangsmaterial zur Herstellung von Arzneimitteln.

Beispiele zur Illustrierung der Erfindung

Beispiel 1

**5,10-Methylen-(6R)-THF•Sulfat**

A. Herstellung von 5,10-Methylen-(6R)-tetrahydrofolsäure

100 g z.B. nach der in der schweizerischen Patentanmeldung Nr. 108 vom 16.01.1991 beschriebenen Methode hergestelltes reines (6S)-Tetrahydrofolsäure•Benzolsulfonat werden in Wasser suspendiert. Durch Zusatz von 1 N Natronlauge wird das pH auf 7,5 gebracht. Nun fügt man 15 ml 37%ige wässrige Formaldehyd-Lösung zu und stellt mit 2 N Schwefelsäure auf pH 2,5. Die gebildete 5,10-Methylen-(6R)-tetrahydrofolsäure fällt aus und wird abfiltriert. Menge: 74 g.

B. Herstellung von 5,10-Methylen-(6R)-tetrahydrofolsäure•Sulfat

74 g 5,10-Methylen-(6R)-tetrahydrofolsäure werden in 370 ml Eisessig gelöst und in eine Lösung von 97 ml 4 N wässriger Schwefelsäure in 200 ml Eisessig eingetropft. Nach einiger Zeit kristallisiert 5,10-Methylen-(6R)-tetrahydrofolsäure•Sulfat der Formel Methylen-(6R)-THF•$H_2SO_4$ aus. Nach Zusatz von 500 ml Ethanol wird das Produkt abfiltriert. Das Produkt lässt sich aus Ameisensäure mit Ethanol oder aus Essigsäure mit Ethanol/$H_2SO_4$ umfällen.

Ausbeute: 71 g; Gehalt 98% (bestimmt mittels HPLC).
$[\alpha]_D^{25}$ = + 120° (c = 1% in DMF)
Löslichkeiten: Methylen-(6R)-THF•$H_2SO_4$ ist ziemlich wenig löslich in Wasser, löslich in siedender 50% Essigsäure und leicht löslich in heissem Eisessig.

Beispiel 2

**5,10-Methylen-(6R)-THF•Sulfat**

20 g nach der schweizerischen Patentanmeldung Nr. 108 vom 16.01.1991 hergestelltes (6S)-Tetrahydrofolsäure•Sulfat werden in Wasser suspendiert. Durch Zusatz von 2 N Natronlauge wird das pH auf 8,6 eingestellt. Nun Setzt man 3,6 ml 37%ige wässrige Formaldehyd-Lösung zu. Nach 15 Minuten wird die erhaltene Lösung in eine Lösung von 100 ml 2 N Schwefelsäure in 100 ml Eisessig eingerührt. Das ausgeschiedene Produkt wird abfiltriert und mit Ethanol gewaschen.
Man erhält auf diese Weise 14 g Titelverbindung.
Gehalt, bestimmt mittels HPLC: 98,7% Methylen-(6R)-THF•$H_2SO_4$.

Beispiel 3

**5,10-Methylen-(6S)-THF•Sulfat**

5 g (6R)-Tetrahydrofolsäure werden in 36 ml 1 N Natronlauge gelöst und bei pH 8 mit 1,5 ml 36%igem Formaldehyd versetzt. Nach 10 Minuten rührt man die erhaltene Reaktionslösung in 20 ml 2 N Schwefelsäure in 25 ml Eisessig. Nach Zusatz von 30 ml Ethanol wird das ausgeschiedene Produkt abfiltriert.
Man erhält 3,8 g Titelverbindung. Gehalt: 96% Methylen-(6S)-THF•Sulfat.

Beispiel 4

**5,10-Methylen-(6R)-THF•Sulfat**

100 g (6S)-Tetrahydrofolsäure werden in warmem Eisessig gelöst. Die Lösung wird mit 20 ml 37%igem Formaldehyd versetzt, klarfiltriert und mit 180 ml 4 N wässriger Schwefelsäure versalzt. Nach Zusatz von 1,5 l Ethanol wird das ausgeschiedene Produkt abfiltriert. Es besteht aus der Titelverbindung.
Gehalt: 97% Methylen-(6R)-THF•$H_2SO_4$.

Beispiel 5

**5,10-Methylen-(6R)-THF•Sulfat**

50 g (6S)-Tetrahydrofolsäure•Sulfat, hergestellt z.B. nach der schweizerischen Patentanmeldung Nr. 108 vom 16.01.91, mit einem Gehalt von 99,8% (6S)-THF•$H_2SO_4$ suspendiert in Wasser, werden durch Zusatz von 200 ml 2 N Natronlauge gelöst und mit 7,5 ml 37%igem Formaldehyd versetzt. Die Reaktionslösung wird in eine Mischung aus 275 ml 2 N Schwefelsäure und 275 ml Eisessig eingetropft. Methylen-(6R)-THF•$H_2SO_4$ kristallisiert aus, wird abfiltriert und mit Ethanol gewaschen.
Ausbeute: 45 g farbloses Produkt.
HPLC-Analyse: 99,6% Methylen-(6R)-THF•$H_2SO_4$.

Beispiel 6

**5,10-Methylen-(6R)-THF•Benzolsulfonat**

27,1 g (6S)-Tetrahydrofolsäure•Benzolsulfonat analog vorher werden in Wasser durch Zusatz von 25,8 ml 5 N Natronlauge in Lösung gebracht. Nun fügt man 3,5 ml 37%ige wässrigen Formaldehyd zu und rührt während 10 Minuten.
Die erhaltene Reaktionslösung wird in eine Lösung von 21,07 g Benzolsulfonsäure in 100 ml Wasser eingetropft. Das ausgeschiedene Produkt wird abfiltriert, wiederholt in kaltem Ethanol aufgeschlämmt und getrocknet.
Man erhält 21 g Titelverbindung.
HPLC-Analyse: 99,5% Methylen-(6R)-THF•Benzolsulfonat.
$[\alpha]_D^{25} = + 102°$ (c = 1% in DMF)

Beispiel 7

**5,10-Methylen-(6R)-THF•Toluol-4-sulfonat**

27,75 g reines (6S)-Tetrahydrofolsäure•Toluol-4-sulfonat werden in Wasser durch Zusatz von Natronlauge in Lösung gebracht, mit 3,5 ml 37%igem Formaldehyd versetzt und die erhaltene Reaktionslösung wird in eine konzentrierte wässrige Lösung von Toluol-4-sulfonsäure eingerührt.
Man erhält 23 g Titelverbindung mit einem Gehalt (HPLC) von 98,6%.

**Patentansprüche**

1. Salze von 5,10-Methylen-(6R)-, (6R,S)- oder (6S)-tetrahydrofolsäure mit Schwefelsäure oder einer Sulfonsäure.

2. 5,10-Methylen-(6R)-, (6R,S)- und (6S)-tetrahydrofolsäure•Sulfat.

3. 5,10-Methylen-(6R)-, (6R,S)- und (6S)-tetrahydrofolsäure•Benzolsulfonat.

4. 5,10-Methylen-(6R)-, (6R,S)- und (6S)-tetrahydrofolsäure•Toluolsulfonat.

5. Methansulfonsäure-, Ethansulfonsäure-, Phenylmethansulfonsäure-, Campher-10-sulfonsäure-, Naphthalin-1-sulfonsäure-, Naphthalin-2-sulfonsäure-, Naphthalin-1,5-disulfonsäure-Additionssalze der 5,10-Methylen-(6R)-, (6R,S)- und (6S)-tetrahydrofolsäure.

6. Verfahren zur Herstellung der in den Ansprüchen 1 bis 5 definierten Salze von 5,10-Methylen-(6R)-, (6R,S)- oder (6S)-tetrahydrofolsäure, dadurch gekennzeichnet, dass die entsprechende diastereomere Form der 5,10-Methylentetrahydrofolsäure mit Schwefelsäure oder einer Sulfonsäure umgesetzt wird.

7. Verfahren nach Anspruch 6, dadurch gekennzeichnet, dass eine in situ hergestellte 5,10-Methylentetrahydrofolsäure eingesetzt wird.

8. Verfahren nach Anspruch 6 oder 7, dadurch gekennzeichnet, dass die Umsetzung und Isolierung in Wasser oder einem Gemisch aus Wasser und einem mit Wasser mischbaren organischen Lösungsmittel durchgeführt werden.

9. Verfahren nach Anspruch 8, dadurch gekennzeichnet, dass als mit Wasser mischbares organisches Lösungsmittel Essigsäure oder ein niedriger organischer Alkohol eingesetzt wird.

10. Verwendung von Salzen der 5,10-Methylen-(6R)-, (6R,S)- oder (6S)-tetrahydrofolsäure mit Schwefelsäure oder einer Sulfonsäure als Bestandteil und/oder als Ausgangsmaterial zur Herstellung von Arzneimitteln.

**Claims**

1. Salts of 5,10-methylene-(6R)-, (6R,S)- or (6S)-tetrahydrofolic acid with sulphuric acid or a sulphonic acid.

2. 5,10-Methylene-(6R)-, (6R,S)- and (6S)-tetrahydrofolic acid sulphate.

3. 5,10-Methylene-(6R)-, (6R,S)- and (6S)-tetrahydrofolic acid benzenesulphonate.

4. 5,10-Methylene-(6R)-, (6R,S)- and (6S)-tetrahydrofolic acid toluenesulphonate.

5. Methanesulphonic acid, ethanesulphonic acid, phenylmethanesulphonic acid, camphor-10-sulphonic acid, naphthalene-1-sulphonic acid, naphthalene-2-sulphonic acid or naphthalene-1,5-disulphonic acid addition salt of 5,10-methylene-(6R)-, (6R,S)- and (6S)-tetrahydrofolic acid.

6. Process for the preparation of the salts of 5,10-methylene-(6R)-, (6R,S)- or (6S)-tetrahydrofolic acid defined in Claims 1 to 5, characterized in that the corresponding diastereomeric form of 5,10-methylene-tetrahydrofolic acid is reacted with sulphuric acid or a sulphonic acid.

7. Process according to Claim 6, characterized in that a 5,10-methylenetetrahydrofolic acid prepared in situ is employed.

8. Process according to Claim 6 or 7, characterized in that the reaction and isolation are carried out in water or a mixture of water and a water-miscible organic solvent.

9. Process according to Claim 8, characterized in that the water-miscible organic solvent employed is acetic acid or a lower organic alcohol.

10. Use of salts of 5,10-methylene-(6R)-, (6R,S)-or (6S)-tetrahydrofolic acid with sulphuric acid or a sulphonic acid as a consistuent and/or as a starting material for the production of medicaments.

**Revendications**

1. Sels de l'acide 5,10-méthylène-(6R)-, (6R,S)- ou (6S)-tétrahydrofolique avec l'acide sulfurique ou avec un acide sulfonique.

2. Sulfate de l'acide 5,10-méthylène-(6R)-, (6R,S)- et (6S)-tétrahydrofolique.

3. Benzènesulfonate de l'acide 5,10-méthylène-(6R)-, (6R,S)- et (6S)-tétrahydrofolique.

4. Toluènesulfonate de l'acide 5,10-méthylène-(6R)-, (6R,S)- et (6S)-tétrahydrofolique.

5. Sels d'addition avec l'acide méthanesulfonique, l'acide éthanesulfonique, l'acide phénylméthanesulfonique, l'acide camphre-10-sulfonique, l'acide naphtalène-1-sulfonique, l'acide naphtalène-2-sulfonique, l'acide naphtalène-1,5-disulfonique, de l'acide 5,10-méthylène-(6R)-, (6R,S)- et (6S)-tétrahydrofolique.

6. Procédé pour préparer les sels, définis dans les revendications 1 à 5, de l'acide 5,10-méthylène-(6R)-, (6R,S)- ou (6S)-tétrahydrofolique, caractérisé en ce qu'on fait réagir la forme diastéréoisomère correspondante de l'acide 5,10-méthylènetétrahydrofolique avec l'acide sulfurique ou avec un acide sulfonique.

7. Procédé selon la revendication 6, caractérisé en ce qu'on utilise un acide 5,10-méthylènetétrahydrofolique préparé in situ.

8. Procédé selon la revendication 6 ou 7, caractérisé en ce que la réaction et l'isolement sont mis en oeuvre dans l'eau ou dans un mélange d'eau et d'un solvant organique miscible à l'eau.

9. Procédé selon la revendication 8, caractérisé en ce qu'on utilise comme solvant organique miscible à l'eau, l'acide acétique ou un alcool organique inférieur.

10. Utilisation de sels de l'acide 5,10-méthylène-(6R)-, (6R,S)- ou (6S)-tétrahydrofolique avec l'acide sulfurique ou avec un acide sulfonique, comme constituant et/ou comme matière de départ pour préparer des médicaments.